# EUROPEAN PATENT APPLICATION

(11) **EP 1 947 108 A1**
(43) Date of publication of application: **23.07.2008**
(21) Application number: 07001143.2
(22) Date of filing: 19.01.2007
(51) Int. Cl.: C07J 9/00, C07J 41/00, A61K 31/575, A61P 1/16

(54) **TGR5 modulators and methods of use thereof**

(71) Applicant: Intercept Pharmaceuticals, Inc., New York, NY 10014 (US)
(72) Inventor: Pellicciari, Roberto, Prof., 06123 Perugia (IT)
(74) Representative: Crump, Julian Richard John

(57) **Abstract**

The invention relates to compounds of Formula I: 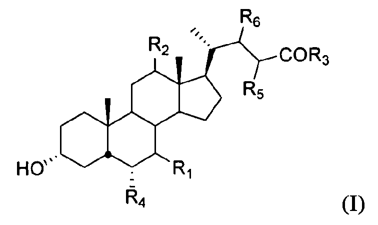 or a pharmaceutically effective salt, solvate, hydrate, or prodrug thereof, wherein: R₁ is hydrogen, hydroxy, or halogen; R₂ is hydrogen or α-hydroxy; R₃ is hydroxy, NH(CH₂)ₘSO₃H, or NH(CH₂)ₙCO₂H; R₄ is hydrogen, alkyl, or halogen; R₅ is unsubstituted or substituted alkyl, or aryl; R₆ is hydrogen or R₅ and R₆ taken together with the carbons to which they are attached form a ring of size 3, 4, 5, or 6 atoms; m is an integer 0, 1, 2, 3, 4, or 5, and n is an integer 0, 1, 2, 3, 4, or 5. The compounds of Formula I are TGR5 modulators useful for the treatment of various diseases.

## Description

### Field of the Invention

The invention concerns relates to compounds that modulate TGR5 and compositions useful in methods for the treatment of various diseases.

### Background of the Invention

TGR5 receptor is a G-protein-coupled receptor that has been identified as a cell-surface receptor that is responsive to bile acids. The primary structure of TGR5 and its responsiveness to bile acids has been found to be highly conserved in TGR5 among human, bovine, rabbit, rat, and mouse, and thus suggests that TGR5 has important physiological functions. TGR5 has been found to be widely distributed in not only lymphoid tissues but also in other tissues. High levels of TGR5 mRNA have been detected in placenta, spleen, and monocytes/macrophages. Bile acids have been shown to induce internalization of the TGR5 fusion protein from the cell membrane to the cytoplasm. Kawamata et al. 2003, J. Bio. Chem., 278, 9435. TGR5 has been found to be identical to hGPCR19 reported by Takeda et al. 2002, FEBS Lett. 520, 97-101.

Bile acids are cholesterol metabolites that are formed in the liver and secreted into the duodenum of the intestine. Bile acids are compounds that play essential roles in the absorption of dietary lipids and regulation of bile acid synthesis. For example, Farnesoid X receptor (FXR) and pregnane X receptor (PXR) have recently been identified as specific nuclear receptors for bile acids. Through activation of FXR, bile acids repress the expression of the rate-limiting enzyme in bile acid synthesis, cholesterol 7a-hydroxylase (Cyp7a). The activation of PXR by bile acids results in both the repression of Cyp7a and the transcriptional induction of the bile acid-metabolizing enzyme, cytochrome P450 3a. At high concentrations, bile acids are also known to exhibit immunosuppressive effects on cell-mediated immunity and macrophage functions. Bile acids including deoxycholic acid (DCA) and chenodeoxycholic acid (CDCA) have been demonstrated to have inhibitory activities on the lipopolysaccharide (LPS)-induced promotion of cytokines in macrophages, including interleukin (IL)-1, IL-6, and tumor necrosis factor alpha (TNFα).

Bile acid compounds that modulate TGR5 have been used for the treatment of various diseases, including central nervous diseases as well as inflammatory diseases (WO 01/77325 and WO 02/84286). Specifically, bile acid compounds alkylated in position 6 of cholanic acid have been disclosed in WO 02/072598, WO 2004/0007521, and EP 1568706 as FXR agonists. The bile acid compound, 23-methyl-ursodeoxycholic (3-alpha,7-beta-dihydroxy-5-beta-cholan-24-oic acid) has also been disclosed (Hepatology, 1988, 8(6), 1571-1576) for the treatment of cholestatic liver diseases.

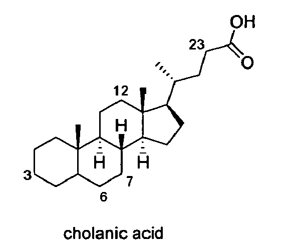

Modulators of TGR5 provide methods of regulating bile acid and cholesterol homeostasis, fatty acid absorption, and protein and carbohydrate digestion. There is a need for the development of TGR5 modulators for the treatment of various diseases. The present invention has identified compounds that modulate TGR5 as well as methods of using these compounds to treat disorders such as central nervous system diseases, inflammatory diseases, and metabolic diseases such as obesity and insulin sensitivity.

### Summary of the Invention

The present invention relates to TGR5 modulators and their use to treat various diseases. In one aspect the invention relates to a compound of formula I: 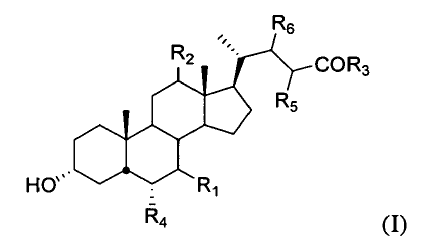 or a pharmaceutically effective salt, solvate, hydrate, or prodrug thereof, wherein R₁ is hydrogen, hydroxy, or halogen; R₂ is hydrogen or α-hydroxy; R₃ is hydroxy, NH(CH₂)ₘSO₃H, or NH(CH₂)ₙCO₂H; R₄ is hydrogen, alkyl, or halogen; R₅ is unsubstituted or substituted alkyl, or aryl; R₆ is hydrogen or R₅ and R₆ taken together with the carbons to which they are attached form a ring of size 3, 4, 5, or 6 atoms; m is an integer 0, 1, 2, 3, 4, or 5; and n is an integer 0, 1, 2, 3, 4, or 5; with the proviso that when R₅ is methyl, R₁ is hydroxyl, and R₃ is hydrogen or NECH₂CH₂SO₃H, then R₄ is not hydrogen.

In one aspect of the present invention, R₁ is hydrogen or hydroxy. R₁ is α-hydroxy. R₁ is β-hydroxy. R₂ is α-hydroxy. R₁ is β-hydroxy and R₂ is α-hydroxy. R₁ is β-hydroxy and R₂ is H. R₁ is α-hydroxy and R₂ is H. R₁ and R₂ are each α-hydroxy. R₁ and R₂ are each hydrogen.

In another aspect of the present invention, R₃ is hydrogen, NH(CH₂)ₘSO₃H, or NH(CH₂)ₙCO₂H. R₃ is hydrogen. R₃ is not hydrogen. R₃ is NH(CH₂)ₘSO₃H. R₃ is NH(CH₂)ₘSO₃H and m is 2. R₃ is NH(CH₂)ₙCO₂H. R₃ is NH(CH₂)ₙCO₂H and n is 1.

In another aspect of the present invention, R₄ is hydrogen or alkyl. R₄ is hydrogen. R₄ is alkyl. R₄ is in the alpha configuration. R₄ is methyl or ethyl. R₃ and R₄ are the same. R₃ and R₄ are different. R₃ and R₄ are each hydrogen.

In another aspect of the present invention, R₃ is NH(CH₂)ₘSO₃H and R₄ is hydrogen. R₃ is NH(CH₂)ₘSO₃H, R₄ is hydrogen and m is 2. R₃ is NH(CH₂)ₙCO₂H and R₄ is hydrogen. R₃ is NH(CH₂)ₙCO₂H and R₄ is hydrogen and n is 1. R₃ is H and R₄ is alkyl. R₃ is H and R₄ is alkyl and alkyl is in the alpha configuration. R₃ is H and R₄ is methyl is in the alpha configuration.

In another aspect of the present invention, R₅ is unsubstituted or substituted alkyl. R₅ is in the S-configuration R₅ is in the R-configuration. R₅ is methyl or ethyl. R₅ is S-methyl. R₅ is R-methyl. R₅ is S-ethyl. R₅ is R-ethyl. R₅ is alkyl substituted with phenyl. R₅ is benzyl. R₅ is S-benzyl. R₅ is R-benzyl. R₅ is aryl. R₅ is phenyl.

In another aspect of the present invention, R₄ and R₅ are each alkyl. R₄ and R₅ are each alkyl and R₅ is in the S-configuration. R₄ and R₅ are each alkyl and R₄ is in the alpha-configuration. R₄ and R₅ are each alkyl and R₁ is α-hydroxy. R₄ and R₅ are each alkyl and R₂ is hydrogen. R₄ and R₅ are each alkyl, R₁ is α-hydroxy, and R₂ is hydrogen.

In another aspect of the present invention, R₁, R₂, R₃, and R₄ are hydrogen. R₂, R₃, and R₄ are hydrogen. R₂ and R₃ are hydrogen.

In another aspect of the present invention, at least one of R₁, R₂, R₃, R₄, or R₅ is hydrogen. At least two of R₁, R₂, R₃, R₄, or R₅ is hydrogen. At least three of R₁, R₂, R₃, R₄, or R₅ is hydrogen. At least four of R₁, R₂, R₃, R₄, or R₅ is hydrogen.

Another aspect of the present invention includes a composition comprising a compound of formula I. 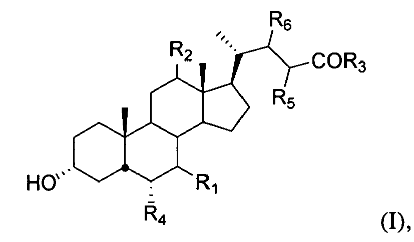 or a salt, solvate, hydrate, or prodrug thereof, and at least one pharmaceutically acceptable excipient wherein R₁ is hydrogen, hydroxy, or halogen; R₂ is hydrogen or α-hydroxy; R₃ is hydroxy, NH(CH₂)ₘSO₃H, or NH(CH₂)ₙCO₂H; R₄ is hydrogen, alkyl, or halogen; R₅ is unsubstituted or substituted alkyl, or aryl; R₆ is hydrogen or R₅ and R₆ taken together with the carbons to which they are attached form a ring of size 3, 4, 5, or 6 atoms; m is an integer 0, 1, 2, 3, 4, or 5; and n is an integer 0, 1, 2, 3, 4, or 5. In one aspect the present invention includes a composition comprising a compound of formula I with the proviso that when R₅ is methyl, R₁ is hydroxyl, and R₃ is hydrogen or NHCH₂CH₂SO₃H, then R₄ is not hydrogen.

Another aspect of the present invention includes a method of treating disease in a subject, comprising administering a therapeutically effective amount of a compound of formula I to a subject in need thereof. The present invention includes treatment of the disease that involves modulation of TGR5 receptor. In one aspect, the disease is a metabolic disease, including obesity and insulin sensitivity. In another aspect, the disease is inflammatory disease, including rheumatoid arthritis and allergy. The present invention includes where the subject is human.

The above description sets forth rather broadly the more important features of the present invention in order that the detailed description thereof that follows may be understood, and in order that the present contributions to the art may be better appreciated. Other objects and features of the present invention will become apparent from the following detailed description considered in conjunction with the examples.

### Description of the Invention

The details of one or more embodiments of the invention are set forth in the accompanying description below. Although any methods and materials similar or equivalent to those described herein can be used in the practice or testing of the present invention, the methods and materials are now described. Other features, objects, and advantages of the invention will be apparent from the description. In the specification, the singular forms also include the plural unless the context clearly dictates otherwise. Unless defined otherwise, all technical and scientific terms used herein have the same meaning as commonly understood by one of ordinary skill in the art to which this invention belongs. In the case of conflict, the present specification will control.

### Definitions

For convenience, certain terms used in the specification, examples and appended claims are collected here.

"Treating", includes any effect, e.g., lessening, reducing, modulating, or eliminating, that results in the improvement of the condition, disease, disorder, etc.

"Alkyl" includes saturated aliphatic groups, including straight-chain alkyl groups (*e.g*., methyl, ethyl, propyl, butyl, pentyl, hexyl, heptyl, octyl, nonyl, decyl), branched-chain alkyl groups (*e.g*., isopropyl, tert-butyl, isobutyl), cycloalkyl (*e.g*., alicyclic) groups (*e.g*., cyclopropyl, cyclopentyl, cyclohexyl, cycloheptyl, cyclooctyl), alkyl substituted cycloalkyl groups, and cycloalkyl substituted alkyl groups. In certain embodiments, a straight chain or branched chain alkyl has six or fewer carbon atoms in its backbone (*e.g*., C₁-C₆ for straight chain, C₃-C₆ for branched chain). In some examples, a straight chain or branched chain alkyl has four or fewer carbon atoms in its backbone. Further, cycloalkyls have from three to eight carbon atoms in their ring structure.

The term "substituted alkyls" refers to alkyl moieties having substituents replacing a hydrogen on at least one carbons of the hydrocarbon backbone. Such substituents can include, for example, alkyl, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkylamino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

"Aryl" includes groups with aromaticity, including 5- and 6-membered "unconjugated", or single-ring, aromatic groups that may include from zero to four heteroatoms, as well as "conjugated", or multicyclic, systems with at least one aromatic ring. Examples of aryl groups include benzene, phenyl, pyrrole, furan, thiophene, thiazole, isothiazole, imidazole, triazole, tetrazole, pyrazole, oxazole, isooxazole, pyridine, pyrazine, pyridazine, and pyrimidine, and the like. Furthermore, the term "aryl" includes multicyclic aryl groups, e.g., tricyclic, bicyclic, e.g., naphthalene, benzoxazole, benzodioxazole, benzothiazole, benzoimidazole, benzothiophene, methylenedioxyphenyl, quinoline, isoquinoline, napthridine, indole, benzofuran, purine, benzofuran, deazapurine, or indolizine. Those aryl groups having heteroatoms in the ring structure may also be referred to as "aryl heterocycles", "heterocycles," "heteroaryls" or "heteroaromatics". The aromatic ring can be substituted at at least one ring position with such substituents as described above, as for example, halogen, hydroxyl, alkoxy, alkylcarbonyloxy, arylcarbonyloxy, alkoxycarbonyloxy, aryloxycarbonyloxy, carboxylate, alkylcarbonyl, alkylaminocarbonyl, aralkylaminocarbonyl, alkenylaminocarbonyl, alkylcarbonyl, arylcarbonyl, aralkylcarbonyl, alkenylcarbonyl, alkoxycarbonyl, aminocarbonyl, alkylthiocarbonyl, phosphate, phosphonato, phosphinato, cyano, amino (including alkylamino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety. Aryl groups can also be fused or bridged with alicyclic or heterocyclic rings, which are not aromatic so as to form a multicyclic system (*e.g*., tetralin, methylenedioxyphenyl).

Unless the number of carbons is otherwise specified, "lower alkyl" includes an alkyl group, as defined above, but having from one to ten, for example, from one to six, carbon atoms in its backbone structure.

The term "alkoxy" or "alkoxyl" includes alkyl, alkenyl, and alkynyl groups covalently linked to an oxygen atom. Examples of alkoxy groups (or alkoxyl radicals) include methoxy, ethoxy, isopropyloxy, propoxy, butoxy, and pentoxy groups.

The term "ether" includes compounds or moieties which contain an oxygen bonded to two different carbon atoms or heteroatoms. For example, the term includes "alkoxyalkyl" which refers to an alkyl, alkenyl, or alkynyl group covalently bonded to an oxygen atom which is covalently bonded to another alkyl group.

The term "ester" includes compounds and moieties which contain a carbon or a heteroatom bound to an oxygen atom which is bonded to the carbon of a carbonyl group. The term "ester" includes alkoxycarboxy groups such as methoxycarbonyl, ethoxycarbonyl, propoxycarbonyl, butoxycarbonyl, pentoxycarbonyl, etc. The alkyl, alkenyl, or alkynyl groups are as defined above.

The term "hydroxy" or "hydroxyl" includes groups with an -OH or -O-.

The term "halogen" includes fluorine, bromine, chlorine, iodine, etc. The term "perhalogenated" generally refers to a moiety wherein all hydrogens are replaced by halogen atoms.

An "anionic group," as used herein, refers to a group that is negatively charged at physiological pH. Anionic groups include carboxylate, sulfate, sulfonate, sulfinate, sulfamate, tetrazolyl, phosphate, phosphonate, phosphinate, or phosphorothioate or functional equivalents thereof. "Functional equivalents" of anionic groups are intended to include bioisosteres, e.g., bioisosteres of a carboxylate group. Bioisosteres encompass both classical bioisosteric equivalents and non-classical bioisosteric equivalents. Classical and non-classical bioisosteres are known in the art (see, *e.g*., Silverman, R. B. The Organic Chemistry of Drug Design and Drug Action, Academic Press, Inc.: San Diego, Calif., 1992, pp. 19-23). Another anionic group is a carboxylate.

The term "unstable functionality" refers to a substitution pattern that contains a labile linkage, *e.g*., a functionality or bond that is susceptible to hydrolysis or cleavage under physiological conditions (e.g., aqueous solutions in the neutral pH range). Examples of unstable functionalities include acetals and ketals.

The terms "crystal polymorphs" or "polymorphs" refer to the existence of more than one crystal form for a compound, salt or solvate thereof. Crystal polymorphs of the bile acid analog compounds are prepared by crystallization under different conditions.

Additionally, the compounds of the present invention, for example, the salts of the compounds, can exist in either hydrated or unhydrated (the anhydrous) form or as solvates with other solvent molecules. Nonlimiting examples of hydrates include monohydrates, dihydrates, etc. Nonlimiting examples of solvates include ethanol solvates, acetone solvates, etc.

"Solvates" means solvent addition forms that contain either stoichiometric or non stoichiometric amounts of solvent. Some compounds have a tendency to trap a fixed molar ratio of solvent molecules in the crystalline solid state, thus forming a solvate. If the solvent is water the solvate formed is a hydrate, when the solvent is alcohol, the solvate formed is an alcoholate. Hydrates are formed by the combination of one or more molecules of water with one of the substances in which the water retains its molecular state as H₂O, such combination being able to form one or more hydrate.

It will be noted that the structure of some of the compounds of the invention include asymmetric carbon atoms. It is to be understood accordingly that the isomers arising from such asymmetry (*e.g*., all enantiomers and diastereomers) are included within the scope of the invention, unless indicated otherwise. Such isomers can be obtained in substantially pure form by classical separation techniques and by stereochemically controlled synthesis. Enantiomers (Rand S-configurations) are named according to the system developed by R.S. Cahn, C. Ingold, and V. Prelog.

Further, the structures and other compounds discussed in this application include all atropic isomers thereof. Atropic isomers are a type of stereoisomer in which the atoms of two isomers are arranged differently in space. Atropic isomers owe their existence to a restricted rotation caused by hindrance of rotation of large groups about a central bond. Such atropic isomers typically exist as a mixture, however as a result of recent advances in chromatography techniques, it has been possible to separate mixtures of two atropic isomers in select cases.

"Stable compound" and "stable structure" are meant to indicate a compound that is sufficiently robust to survive isolation to a useful degree of purity from a reaction mixture, and formulation into an efficacious therapeutic agent.

As used herein, the term "analog" refers to a chemical compound that is structurally similar to another but differs slightly in composition (as in the replacement of one atom by an atom of a different element or in the presence of a particular functional group, or the replacement of one functional group by another functional group). Thus, an analog is a compound that is similar or comparable in function and appearance, but not in structure or origin to the reference compound.

As defined herein, the term "derivative", *e.g*., in the term "bile acid derivatives", refers to compounds that have a common core structure, and are substituted with various groups as described herein.

The term "bioisostere" refers to a compound resulting from the exchange of an atom or of a group of atoms with another, broadly similar, atom or group of atoms. The objective of a bioisosteric replacement is to create a new compound with similar biological properties to the parent compound. The bioisosteric replacement may be physicochemically or topologically based. Examples of carboxylic acid bioisosteres include acyl sulfonimides, tetrazoles, sulfonates, and phosphonates. *See, e.g.,* Patani and LaVoie, Chem. Rev. 96, 3147-3176 (1996).

"Combination therapy" (or "co-therapy") includes the administration of a compound of the invention and at least a second agent as part of a specific treatment regimen intended to provide the beneficial effect from the co-action of these therapeutic agents. The beneficial effect of the combination includes, but is not limited to, pharmacokinetic or pharmacodynamic co-action resulting from the combination of therapeutic agents. Administration of these therapeutic agents in combination typically is carried out over a defined time period (usually minutes, hours, days or weeks depending upon the combination selected). "Combination therapy" may, but generally is not, intended to encompass the administration of two or more of these therapeutic agents as part of separate monotherapy regimens that incidentally and arbitrarily result in the combinations of the present invention. "Combination therapy" is intended to embrace administration of these therapeutic agents in a sequential manner, that is, wherein each therapeutic agent is administered at a different time, as well as administration of these therapeutic agents, or at least two of the therapeutic agents, in a substantially simultaneous manner. Substantially simultaneous administration can be accomplished, for example, by administering to the subject a single capsule having a fixed ratio of each therapeutic agent or in multiple, single capsules for each of the therapeutic agents. Sequential or substantially simultaneous administration of each therapeutic agent can be effected by any appropriate route including, but not limited to, oral routes, intravenous routes, intramuscular routes, and direct absorption through mucous membrane tissues. The therapeutic agents can be administered by the same route or by different routes. For example, a first therapeutic agent of the combination selected may be administered by intravenous injection while the other therapeutic agents of the combination may be administered orally. Alternatively, for example, all therapeutic agents may be administered orally or all therapeutic agents may be administered by intravenous injection. The sequence in which the therapeutic agents are administered is not narrowly critical.

"Combination therapy" also embraces the administration of the therapeutic agents as described above in further combination with other biologically active ingredients and non-drug therapies (*e.g*., surgery or mechanical treatments). Where the combination therapy further comprises a non-drug treatment, the non-drug treatment may be conducted at any suitable time so long as a beneficial effect from the co-action of the combination of the therapeutic agents and non-drug treatment is achieved. For example, in appropriate cases, the beneficial effect is still achieved when the non-drug treatment is temporally removed from the administration of the therapeutic agents, perhaps by days or even weeks.

The terms "parenteral administration" and "administered parenterally" as used herein refer to modes of administration other than enteral and topical administration, usually by injection, and includes, without limitation, intravenous, intramuscular, intra-arterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

The term "pulmonary" as used herein refers to any part, tissue or organ whose primary function is gas exchange with the external environment, *e.g*., O₂/CO₂ exchange, within a patient. "Pulmonary" typically refers to the tissues of the respiratory tract. Thus, the phrase "pulmonary administration" refers to administering the formulations described herein to any part, tissue or organ whose primary function is gas exchange with the external environment (*e.g*., mouth, nose, pharynx, oropharynx, laryngopharynx, larynx, trachea, carina, bronchi, bronchioles, alveoli). For purposes of the present invention, "pulmonary" also includes a tissue or cavity that is contingent to the respiratory tract, in particular, the sinuses.

An "effective amount" of a compound of the disclosed invention is the quantity which, when administered to a subject in need of treatment, ameliorates symptoms arising from the disease. The amount of the disclosed compound to be administered to a subject will depend on the particular disorder, the mode of administration, co-administered compounds, if any, and the characteristics of the subject, such as general health, other diseases, age, sex, genotype, body weight and tolerance to drugs. The skilled artisan will be able to determine appropriate dosages depending on these and other factors.

A "pharmaceutically acceptable salt" or "salt" of the disclosed compound is a product of the disclosed compound that contains an ionic bond, and is typically produced by reacting the disclosed compound with either an acid or a base, suitable for administering to a subject.

A "composition" is a formulation containing the disclosed compounds in a form suitable for administration to a subject. In another embodiment, the pharmaceutical composition is in bulk or in unit dosage form. The unit dosage form is any of a variety of forms, including, for example, a capsule, an IV bag, a tablet, a single pump on an aerosol inhaler, or a vial. The quantity of active ingredient (*e.g*., a formulation of the disclosed compound or salts thereof) in a unit dose of composition is an effective amount and is varied according to the particular treatment involved. One skilled in the art will appreciate that it is sometimes necessary to make routine variations to the dosage depending on the age and condition of the patient. The dosage will also depend on the route of administration. A variety of routes are contemplated, including oral, pulmonary, rectal, parenteral, transdermal, subcutaneous, intravenous, intramuscular, intraperitoneal, intranasal, and the like. Dosage forms for the topical or transdermal administration of a compound of this invention include powders, sprays, ointments, pastes, creams, lotions, gels, solutions, patches and inhalants. In another embodiment, the active compound is mixed under sterile conditions with a pharmaceutically acceptable carrier, and with any preservatives, buffers, or propellants that are required.

The term "flash dose" refers to compound formulations that are rapidly dispersing dosage forms.

The term "immediate release" is defined as a release of compound from a dosage form in a relatively brief period of time, generally up to about 60 minutes. The term "modified release" is defined to include delayed release, extended release, and pulsed release. The term "pulsed release" is defined as a series of releases of drug from a dosage form. The term "sustained release" or "extended release" is defined as continuous release of a compound from a dosage form over a prolonged period.

A "subject" includes mammals, *e.g*., humans, companion animals (*e.g*., dogs, cats, birds, and the like), farm animals (*e.g*., cows, sheep, pigs, horses, fowl, and the like) and laboratory animals (*e.g*., rats, mice, guinea pigs, birds, and the like). Typically, the subject is human.

Compounds of the invention also include prodrugs or physiologically equivalent derivatives. A "prodrug" or "physiologically equivalent derivative" includes a precursor form of the drug which is metabolically converted *in vivo* to produce the active drug. The invention further contemplates the use of prodrugs which are converted in vivo to the TGR5 modulating compounds used in the methods of the invention (*see, e.g.,* R. B. Silverman, 1992, "The Organic Chemistry of Drug Design and Drug Action", Academic Press, Chp. 8). Such prodrugs can be used to alter the biodistribution (*e.g*., to allow compounds which would not typically cross the blood-brain barrier to cross the blood-brain barrier) or the pharmacokinetics of the TGR5 modulating compound. For example, an anionic group, *e.g*., a carboxylate, sulfate or sulfonate, can be esterified, *e.g.*, with an alkyl group (*e.g*., a methyl group) or a phenyl group, to yield an ester. When the ester is administered to a subject, the ester is cleaved, enzymatically or non-enzymatically, reductively or hydrolytically, to reveal the anionic group. Such an ester can be cyclic, *e.g*., a cyclic sulfate or sulfone, or two or more anionic moieties may be esterified through a linking group. An anionic group can be esterified with moieties (*e.g*., acyloxymethyl esters) which are cleaved to reveal an intermediate TGR5 modulating compound which subsequently decomposes to yield the active TGR5 modulating compound. In one embodiment, the prodrug is a reduced form of a carboxylate, sulfate or sulfonate, *e*.*g*., an alcohol or thiol, which is oxidized in vivo to the TGR5 modulating compound. Furthermore, an anionic moiety can be esterified to a group which is actively transported *in vivo,* or which is selectively taken up by target organs.

As used herein, the term "amino acid conjugates" refers to conjugates of the compounds of formula I with any suitable amino acid. Taurine (NH(CH₂)₂SO₃H) and glycine (NHCH₂CO₂H) are examples of amino acid conjugates. Suitable amino acid conjugates of the compounds of formula I have the added advantage of enhanced integrity in bile or intestinal fluids. Suitable amino acids are not limited to taurine and glycine. The present invention encompasses amino acid conjugates of the compounds of formula **I.**

The term "TGR5 modulator" means any compound that interacts with the TGR5 receptor. The interaction is not limited to a compound acting as an antagonist, agonist, partial agonist, or inverse agonist of the TGR5 receptor. The profile of a ligand, traditionally, endogenous or synthetic, is characterized by its intrinsic efficacy 'e' originally described by Furchgott in 1966. It is used to express the degree to which the different ligands produce varying biological responses while occupying the same number of receptors. Generally, the term "agonist" means a compound that enhances the activity of another molecule or receptor site. An agonist, by classical definition, whether a orthosteric, allosteric, inverse or a co-agonist has a property to bind to the receptor, alter its receptor state and result in a biological action. Consequently, agonism is defined as a property of an agonist or a ligand to produce a biological action. In contrast to this, an "antagonist" is essentially an agonist with high affinity to the same receptor macromolecule, but with very less or negligible intrinsic efficacy, and thus sterically prevents the biological actions of an agonist. As a property, antagonism may be functional or physiological, where an agonist has a direct competition for the receptor site in former and opposing effects via a different receptor-messenger system in the later. More specifically, a TGR5 agonist is a receptor ligand or compound that binds to TGR5 and increases the concentration of cyclic adenosine monophosphate (cAMP) by at least 20% in cells expressing the receptor." Conversely, a TGR5 antagonist would be a compound that antagonizes or blocks the activity of an agonist, thereby effecting a reduction in the concentration of cAMP

The term "metabolic disorders" includes but is not limited to dyslipidemia, atherosclerosis, obesity, coronary heart disease, stroke, insulin resistance/sensitivity, and diabetes.

The term "inflammatory disease" means an inflammatory response which causes injury to autologous tissues. Inflammatory diseases include but are not limited to rheumatoid arthritis, osteoarthritis, cervical spondylosis, cumulative trauma disorder, allergy, endometriosis, pelvic inflammatory disease, adhesive peritonitis, appendicitis, pericarditis, and pleuritis.

The present invention relates to compounds having TGR5 receptor modulating activity and their use to treat various diseases including, central nervous system diseases, inflammatory diseases, and metabolic diseases such as obesity and insulin sensitivity. According to the first aspect, the present invention provides a compound of formula I: 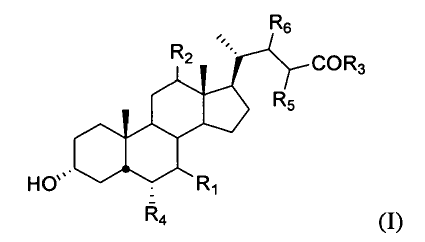 or a pharmaceutically effective salt, solvate, hydrate, or prodrug thereof, wherein: R₁ is hydrogen, hydroxy, or halogen; R₂ is hydrogen or α-hydroxy; R₃ is hydroxy, NH(CH₂)ₘSO₃H, or NH(CH₂)ₙCO₂H; R₄ is hydrogen, alkyl, or halogen; R₅ is unsubstituted or substituted alkyl, or aryl; R₆ is hydrogen or R₅ and R₆ taken together with the carbons to which they are attached form a ring of size 3, 4, 5, or 6 atoms; m is an integer 0, 1, 2, 3, 4, or 5, and n is an integer 0, 1, 2, 3, 4, or 5; with the proviso that when R₅ is methyl, R₁ is hydroxyl, and R₃ is hydrogen or NHCH₂CH₂SO₃H, then R₄ is not hydrogen.

In one aspect, the present invention provides compounds where R₁ is hydrogen or hydroxy. R₁ is hydroxy. R₁ is hydrogen. R₁ is α-hydroxy. R₁ is β-hydroxy.

In another aspect, the present invention provides compounds where R₁ is halogen. R₁ is fluorine. R₁ is α-fluorine. R₁ is β-fluorine. The stereochemistry of R₁ in the α- and β-configurations is shown below:

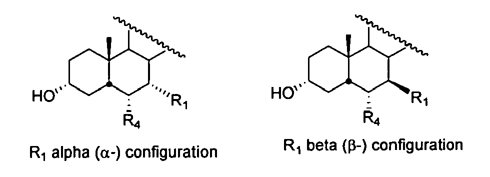

In another aspect, the present invention provides compounds where R₂ is α-hydroxy. R₂ is hydrogen. R₁ is β-hydroxy and R₂ is α-hydroxy. R₁ is β-hydroxy and R₂ is H. R₁ is α-hydroxy and R₂ is H.

In another aspect, the present invention provides compounds where at least one of R₁ or R₂ is hydroxy. In another aspect, at least one of R₁ or R₂ is hydrogen. R₁ and R₂ are the same. R₁ and R₂ are each α-hydroxy. R₁ and R₂ are each hydrogen.

In another aspect, the present invention provides compounds where R₃ is hydrogen, NH(CH₂)ₘSO₃H, or NH(CH₂)ₙCO₂H. R₃ is hydrogen. R₃ is not hydrogen. R₃ is NH(CH₂)ₘSO₃H. In another aspect, R₃ is NH(CH₂)ₘSO₃H and m is 2. R₃ is NH(CH₂)ₙCO₂H. In another aspect, R₃ is NH(CH₂)ₙCO₂H and n is 1.

In another aspect, R₄ is hydrogen or alkyl. R₄ is hydrogen. R₄ is lower alkyl. R₄ is lower alkyl and the lower alkyl group is in the alpha configuration. R₄ in the alpha configuration means that R₄ has the stereochemistry shown in the structure below.

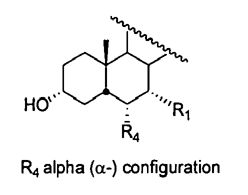

In another aspect, R₄ is halogen. R₄ is fluorine. R₄ is halogen and the halogen is in the alpha configuration. R₄ is α-fluorine.

In another aspect, R₄ is methyl or ethyl. R₄ is methyl. R₄ is ethyl. R₄ is α-methyl. R₄ is α-ethyl. R₃ and R₄ are the same. R₃ and R₄ are different. R₃ and R₄ are each hydrogen. R₃ is NH(CH₂)ₘSO₃H and R₄ is hydrogen. In another aspect, R₃ is NH(CH₂)ₘSO₃H, R₄ is hydrogen and m is 2. R₃ is NH(CH₂)ₙCO₂H and R₄ is hydrogen. In another aspect, R₃ is NH(CH₂)ₙCO₂H, R₄ is hydrogen and n is 1.

In another aspect, R₃ is H and R₄ is alkyl. R₃ is H and R₄ is lower alkyl. Lower alkyl is in the alpha configuration. R₃ is H and R₄ is methyl. R₃ is H and R₄ is ethyl. R₃ is H and R₄ is α-methyl. R₃ is H and R₄ is α-ethyl.

In another aspect, R₅ is not hydrogen. R₅ is unsubstituted or substituted alkyl. R₅ is unsubstituted or substituted lower alkyl. R₅ is in the S-configuration. R₅ is in the R-configuration. R₅ is methyl or ethyl. R₅ is S-methyl. R-methyl. R₅ is S-ethyl. R-ethyl. R₅ is alkyl substituted with phenyl. R₅ is lower alkyl substituted with phenyl. R₅ is benzyl. R₅ is S-benzyl. R₅ is R-benzyl.

In another aspect, R₅ is aryl. R₅ is phenyl.

In another aspect, R₄ and R₅ are each alkyl. R₄ and R₅ are each lower alkyl. R₄ and R₅ are each lower alkyl and R₅ is in the S-configuration. R₄ and R₅ are each lower alkyl and R₄ is in the alpha configuration. In another aspect, R₄ and R₅ are not hydrogen.

In another aspect, R₄ and R₅ are each lower alkyl and R₁ is α-hydroxy. R₄ and R₅ are each lower alkyl and R₂ is hydrogen. R₄ and R₅ are each lower alkyl, R₁ is α-hydroxy, and R₂ is hydrogen.

In another aspect, R₅ and R₆ taken together with the carbons to which they are attached form a ring size of 3, 4, 5, or 6 atoms. R₅ and R₆ taken together with the carbons to which they are attached form a 3-membered ring. The 3-membered ring has the following stereochemistry: 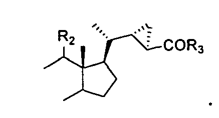 The 3-membered ring has the following stereochemistry:

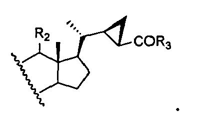

In another aspect, R₁, R₂, R₃, and R₄ are hydrogen. R₂, R₃, and R₄ are hydrogen. R₂ and R₃ are hydrogen.

In another aspect, at least one of R₁, R₂, R₃, R₄, or R₅ is hydrogen.

In another aspect, at least two of R₁, R₂, R₃, R₄, or R₅ is hydrogen.

In another aspect, at least three of R₁, R₂, R₃, R₄, or R₅ is hydrogen.

In another aspect, at least four of R₁, R₂, R₃, R₄, or R₅ is hydrogen.

In another aspect, the compound does not include when R₅ is methyl, R₄ is hydrogen, and R₂ is H or OH.

In another aspect of the present invention, the compound is selected from Compounds **Ia, Ib, Ic, Ig, Ih, Ii, Io, Ip, Iq, Ia1, Ib1, Ic1, Ig1, Ih1, Iil, Il1, Im1, In1, Io1, Ip1, Iq1, Ia2, Ib2, Ic2, Id2, Ie2, If2, Ig2, Ih2, Ii2, I12, Im2, In2, Io2, Ip2, Iq2, Ia3, Ib3, Ic3, Id3, Ie3, If3, Ig3, Ih3, Ii3, Il3, Im3, In3, Ia4, Ib4, Ic4, Id4, Ie4, If4, Ig4, Ih4, Ii4, Il4, Im4, In4, Ia5, Ib5, Ic5, Id5, Ie5, If5, Ig5, Ih5, Ii5, Il5, Im5 or In5.**

In another aspect of the present invention, the compound is not selected from Compounds **Id, Ie, If, Id1, Il, Im, or In.**

In another aspect of the present invention, the compounds are modulate the activity of TGR5 receptor. The present invention includes compounds that are TGR5 receptor agonists. In one aspect, the present invention includes compounds that are highly selective for the TGR5 receptor over the FXR receptor.

Another aspect of the present invention includes a composition comprising a compound of formula I: 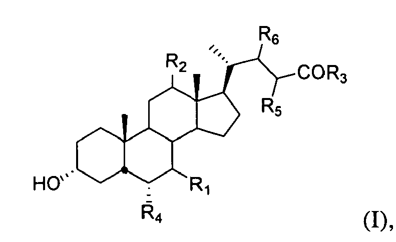 or a salt, solvate, hydrate, or prodrug thereof, and at least one pharmaceutically acceptable excipient wherein R₁ is hydrogen, hydroxy, or halogen; R₂ is hydrogen or α-hydroxy; R₃ is hydroxy, NH(CH₂)ₘSO₃H, or NH(CH₂)ₙCO₂H; R₄ is hydrogen, alkyl, or halogen; R₅ is unsubstituted or substituted lower alkyl, or aryl; R₆ is hydrogen or R₅ and R₆ taken together with the carbons to which they are attached form a ring of size 3, 4, 5, or 6 atoms; m is an integer 0, 1, 2, 3, 4, or 5; and n is an integer 0, 1, 2, 3, 4, or 5. In another aspect, the present invention includes a composition comprising a compound of formula I with proviso that when R₅ is methyl, R₁ is hydroxyl, and R₃ is hydrogen or NHCH₂CH₂SO₃H, then R₄ is not hydrogen.

Another aspect of the invention includes a method of treating disease in a subject, comprising administering a therapeutically effective amount of a compound of formula I to a subject in need thereof: 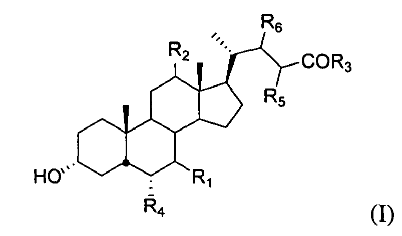 or a pharmaceutically effective salt, solvate, hydrate, or prodrug thereof, wherein: R₁ is hydrogen, hydroxy, or halogen; R₂ is hydrogen or α-hydroxy;
R₃ is hydroxy, NH(CH₂)ₘSO₃H, or NH(CH₂)ₙCO₂H; R₄ is hydrogen, alkyl, or halogen;
R₅ is unsubstituted or substituted alkyl, or aryl; R₆ is hydrogen or R₅ and R₆ taken together with the carbons to which they are attached form a ring of size 3, 4, 5, or 6 atoms; m is an integer 0, 1, 2, 3, 4, or 5; and n is an integer 0, 1, 2, 3, 4, or 5. The present invention includes a method for treatment or prevention of a disease or condition that involves modulation of the TGR5 receptor.

The present invention includes a method comprising administerining a therapeutically effective amount of a compound of formula I.

In one aspect of the present invention, the compound is a regulator of a physiological function in which TGR5 is involved, or an agent for the prophylaxis or treatment of pathology or disease in which TGR5 is involved. In another aspect, the compound is a cytokine production suppressor. In another aspect, the compound is a GLP-1 secretion promoter or an insulin secretagoue. In another aspect, the compound is an anorectic agent, a pancreatic regenerator, a pancreatic β cell differentiation promoter, a pancreatic β cell growth promoter or an insulin sensitizer. In another aspect, the compound is an agent for the prophylaxis or treatment of cardiac failure, cardiac infaction, acute kidney failure, angina pectoris, arrhythmia, bronchial asthma, chronic obstructive pulmonary disease, arteriosclerosis, rheumatoid arthritis, diabetes, obesity, insulin hyposecretion, pancreatic fatigue, gastric ulcer, ulcerative colitis, allergy, osteoarthritis, erythematosus, excessive immune reaction after transplantation or infectious disease, or an immunosuppressant.

The present invention includes a method of treatment of a subject affected by a disease wherein the TGR5 receptor is involved, which method includes administration to a subject an effective amount of a compound of formula I.

In another aspect, the invention includes a method for treatment or prevention of a metabolic disease by administering a compound of the invention. In one aspect, the metabolic disease is obesity. In another aspect, the metabolic disease is insulin sensitivity. In another aspect, the metabolic disease is diabetes. In another aspect, the metabolic disease is insulin hyposecretion. In another aspect, the metabolic disease is pancreatic fatigue.

The present invention includes a method for treatment or prevention of an inflammatory disease by administering a compound of the invention. In one aspect, the inflammatory disease is rheumatoid arthritis. In another aspect, the inflammatory disease is allergy.

The present invention includes the subject is human.

The present invention includes use of the compounds of the invention for known traditional uses of the bile acids. Traditional uses of the bile acids include treatment of cholelithiasis, bile desaturation, cholesterol metabolism, and use as an antioxidant, radical scavenger, anticholestatic, enduretic, anti-dyslipemic, and hepatocycle protector.

In another aspect, the method comprises administering a compound of formula I where if R₅ is methyl; R₁ is hydroxy; R₂ is hydrogen or NHCH₂CH₂SO₃H, then R₄ is not hydrogen.

In another aspect, the method includes administering a compound selected from Compounds **Ia, Ib, Ic, Id, Ie, If, Ig, Ih, Ii, Il, Im, In, Io, Ip, Iq, Ia1, Ib1, Ic1, Id1, Ie1, If1 Ig1, Ih1, Ii1, Il1, Im1, In1, Io1, Ip1, Iq1, Ia2, Ib2, Ic2, Id2, Ie2, If2, Ig2, Ih2, Ii2, Il2, Im2, In2, Io2, Ip2, Iq2, Ia3, Ib3, Ic3, Id3, Ie3, If3, Ig3, Ih3, Ii3, Il3, Im3, In3, Ia4, Ib4, Ic4, Id4, Ie4, If4, Ig4, Ih4, Ii4, Il4, Im4, In4, Ia5, Ib5, Ic5, Id5, Ie5, If5, Ig5, Ih5, Ii5, Il5, Im5 or In5.**

In another aspect, the method includes administering a compound selected from **Compounds Ia, Ib, Ic, Ig, Ih, Ii, Io, Ip, Iq, Ia1, Ib1, Ic1, Ig1, Ih1, Iil, Il1, Im1, In1, Io1, Ip1, Iq1, Ia2, Ib2, Ic2, Id2, Ie2, If2, Ig2, Ih2, Ii2, Il2, Im2, In2, Io2, Ip2, Iq2, Ia3, Ib3, Ic3, Id3, Ie3, If3, Ig3, Ih3, Ii3, Il3, Im3, In3, Ia4, Ib4, Ic4, Id4, Ie4, If4, Ig4, Ih4, Ii4, Il4, Im4, In4, Ia5, Ib5, Ic5, Id5, Ie5, If5, Ig5, Ih5, Ii5, Il5, Im5 or In5.**

In one aspect, the method of the present invention does not include administering a compound selected from Compounds **Id, Ie, If, Id1, Il, Im,** or **In.**

Another aspect of the present invention is the use of a compound of formula I: 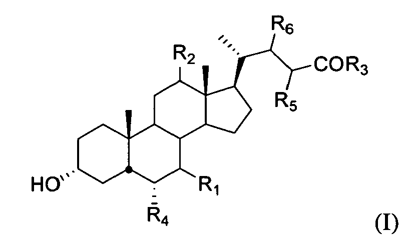 or a salt, solvate, hydrate, or prodrug thereof, and at least one pharmaceutically acceptable excipient wherein R₁ is hydrogen, hydroxy, or halogen; R₂ is hydrogen or α-hydroxy; R₃ is hydroxy, NH(CH₂)ₘSO₃H, or NH(CH₂)ₙCO₂H; R₄ is hydrogen, alkyl, or halogen; R₅ is unsubstituted or substituted lower alkyl, or aryl; R₆ is hydrogen or R₅ and R₆ taken together with the carbons to which they are attached form a ring of size 3, 4, 5, or 6 atoms; m is an integer 0, 1, 2, 3, 4, or 5; and n is an integer 0, 1, 2, 3, 4, or 5 for the preparation of a medicament for the treatment of disease wherein a modulation of TGR5 is desired. In another aspect, the present invention includes a use for the preparation of a medicament for the treatment of disease wherein a modulation of TGR5 is desired comprising a compound of formula I with proviso that when R₅ is methyl, R₁ is hydroxyl, and R₃ is hydrogen or NHCH₂CH₂SO₃H, then R₄ is not hydrogen.

The present invention also provides radiolabeled compounds of formula I. Radiolabeled compounds of formula I can be prepared using conventional techniques. For example, radiolabeled compounds of formula I can be prepared by reacting the compound of formula I with tritium gas in the presence of an appropriate catalyst to produce radiolabeled compounds of formula I. In one embodiment, the compounds of formula I are tritiated.
Some representative compounds of the invention are shown below. For all of the compounds listed below R₆ = H.
**Ia:** R₁= α-OH, R₂= H, R₃= H, R₄= H, R₅= (*S*,*R*)Me
**Ib:** R₁= α-OH, R₂= H, R₃= H, R₄= H, R₅= (*S*)Me
**Ic:** R₁= α-OH, R₂= H, R₃= H, R₄= H, R₅= (*R*)Me
**Id:** R₁= β-OH, R₂= H, R₃= H, R₄= H, R₅= (*S,R*)Me
**Ie:** R₁= β-OH, R₂= H, R₃= H, R₄= H, R₅= (*S*)Me
**If:** R₁= β-OH, R₂= H, R₃= H, R₄=H, R₅= (*R*)Me
**Ig:** R₁= α-OH, R₂= α-OH, R₃= H, R₄= H, R₅= (*S*,*R*)Me
**Ih:** R₁= α-OH, R₂= α-OH, R₃= H, R₄= H, R₅= (*S*)Me
**Ii:** R₁= α-OH, R₂= α-OH, R₃= H, R₄= H, R₅= (*R*)Me
**Il:** R₁= β-OH, R₂= α-OH, R₃= H, R₄= H, R₅= (*S*,*R*)Me
**Im:** R₁= β-OH, R₂= α-OH, R₃= H, R₄= H, R₅= (*S*)Me
**In:** R₁= β-OH, R₂= α-OH, R₃= H, R₄= H, R₅= (*R*)Me
**Io:** R₁= H, R₂= H, R₃= H, R₄= H, R₅= (*S,R*)Me
**Ip:** R₁= H, R₂= H, R₃= H, R₄= H, R₅= (*S*)Me
**Iq:** R₁= H, R₂= H, R₃= H, R₄= H, R₅= (*R*)Me
**Ia1:** R₁= α-OH, R₂= H, R₃= NHCH₂CH₂SO₃H, R₄= H, R₅= (*S,R*)Me
**Ib1:** R₁= α-OH, R₂= H, R₃= NHCH₂CH₂SO₃H, R₄= H, R₅= (*S*)Me
**Ic1:** R₁= α-OH, R₂= H, R₃= NHCH₂CH₂SO₃H, R₄= H, R₅= (*R*)Me
**Id1:** R₁= β-OH, R₂= H, R₃= NHCH₂CH₂SO₃H, R₄= H, R₅= (*S*,*R*)Me
**Ie1:** R₁= β-OH, R₂= H, R₃= NHCH₂CH₂SO₃H, R₄= H, R₅= (*S*)Me
**If1:** R₁= β-OH, R₂= H, R₃= NECH₂CH₂SO₃H, R₄=H, R₅= (*R*)Me
**Ig1:** R₁= α-OH, R₂= α-OH, R₃= NECH₂CH₂SO₃H, R₄= H, R₅= (*S*,*R*)Me
**Ih1:** R₁= α-OH, R₂= α-OH, R₃= NHCH₂CH₂SO₃H, R₄= H, R₅= (*S*)Me
**Ii1:** R₁= α-OH, R₂= α-OH, R₃= NHCH₂CH₂SO₃H, R₄= H, R₅= (*R*)Me
**Il1:** R₁=β-OH, R₂=α-OH, R₃=NHCH₂CH₂SO₃H, R₄=H, R₅=(*S*,*R*)Me
**Im1:** R₁=β-OH, R₂=α-OH, R₃=NHCH₂CH₂SO₃H, R₄=H, R₅=(*S*)Me
**In1:** R₁=β-OH, R₂=α-OH, R₃=NHCH₂CH₂SO₃H, R₄=H, R₅=(*R*)Me
**Io1:** R₁=H, R₂=H, R₃= NHCH₂CH₂SO₃H, R₄=H, R₅=(*S,R*)Me
**Ip1:** R₁=H, R₂=H, R₃=NHCH₂CH₂SO₃H, R₄=H, R₅=(*S*)Me
**Iq1:** R₁=H, R₂=H, R₃=NHCH₂CH₂SO₃H, R₄=H, R₅=(*R*)Me
**Ia2:** R₁=α-OH, R₂=H, R₃=NHCH₂CO₂H, R₄=H, R₅=(*S*,*R*)Me
**Ib2:** R₁=α-OH, R₂=H, R₃=NHCH₂CO₂H, R₄=H, R₅=(*S*)Me
**Ic2:** R₁=α-OH, R₂=H, R₃=NHCH₂CO₂H, R₄=H, R₅=(*R*)Me
**Id2:** R₁=β-OH, R₂=H, R₃=NHCH₂CO₂H, R₄=H, R₅=(*S*,*R*)Me
**Ie2:** R₁= β-OH, R₂= H, R₃= NHCH₂CO₂H, R₄= H, R₅= (*S*)Me
**If2:** R₁= β-OH, R₂= H, R₃= NHCH₂CO₂H, R₄=H, R₅= (*R*)Me
**Ig2:** R₁= α-OH, R₂= α-OH, R₃= NHCH₂CO₂H, R₄= H, R₅= *(S,R)Me*
**Ih2:** R₁= α-OH, R₂= α-OH, R₃= NHCH₂CO₂H, R₄= H, R₅= (*S*)Me
**Ii2:** R₁= α-OH, R₂= α-OH, R₃= NHCH₂CO₂H, R₄= H, R₅= (*R*)Me
**Il2:** R₁= β-OH, R₂= α-OH, R₃= NHCH₂CO₂H, R₄= H, R₅= (*S,R*)Me
**Im2:** R₁= β-OH, R₂= α-OH, R₃= NHCH₂CO₂H, R₄= H, R₅= (*S*)Me
**In2:** R₁= β-OH, R₂= α-OH, R₃= NHCH₂CO₂H, R₄= H, R₅= (*R*)Me
**Io2:** R₁= H, R₂= H, R₃= NHCH₂CO₂H, R₄= H, R₅= (*S,R*)Me
**Ip2:** R₁= H, R₂= H, R₃= NHCH₂CO₂H, R₄= H, R₅= (*S*)Me
**Iq2:** R₁= H, R₂= H, R₃= NHCH₂CO₂H, R₄= H, R₅= (*R*)Me
**Ia3:** R₁= α-OH, R₂= H, R₃= H, R₄= α-Me, R₅= (*S,R*)Me
**Ib3:** R₁= α-OH, R₂= H, R₃= H, R₄= α-Me, R₅= (*S*)Me
**Ic3 :** R₁= α-OH, R₂= H, R₃= H, R₄= α-Me, R₅= (*R*)Me
**Id3:** R₁= β-OH, R₂= H, R₃= H, R₄= α-Me, R₅= (*S,R*)Me
**Ie3:** R₁= β-OH, R₂= H, R₃= H, R₄= α-Me, R₅= (*S*)Me
**If3:** R₁= β-OH, R₂= H, R₃= H, R₄= α-Me, R₅= (*R*)Me
**Ig3:** R₁= α-OH, R₂= α-OH, R₃= H, R₄= α-Me, R₅= (*S,R*)Me
**Ih3:** R₁= α-OH, R₂= α-OH, R₃= H, R₄= α-Me, R₅= (*S*)Me
**Ii3:** R₁= α-OH, R₂= α-OH, R₃= H, R₄= α-Me, R₅= (*R*)Me
**Il3 :** R₁= β-OH, R₂= α-OH, R₃= H, R₄= α-Me, R₅= (*S,R*)Me
**Im3:** R₁= β-OH, R₂= α-OH, R₃= H, R₄= α-Me, R₅= (*S*)Me
**In3 :** R₁= β-OH, R₂= α-OH, R₃= H, R₄= α-Me, R₅= (*R*)Me
**Ia4:** R₁= α-OH, R₂= H, R₃= NHCH₂CH₂SO₃H, R₄= α-Me, R₅= (*S,R*)Me
**Ib4:** R₁= α-OH, R₂= H, R₃= NHCH₂CH₂SO₃H, R₄= α-Me, R₅= (*S*)Me
**Ic4:** R₁= α-OH, R₂= H, R₃= NHCH₂CH₂SO₃H, R₄= α-Me, R₅= (*R*)Me
**Id4:** R₁= β-OH, R₂= H, R₃= NHCH₂CH₂SO₃H, R₄= α-Me, R₅= (*S,R*)Me
**Ie4:** R₁= β-OH, R₂= H, R₃= NHCH₂CH₂SO₃H, R₄= α-Me, R₅= (*S*)Me
**If4:** R₁= β-OH, R₂= H, R₃= NHCH₂CH₂SO₃H, R₄= α-Me, R₅= (*R*)Me
**Ig4:** R₁= α-OH, R₂= α-OH, R₃= NHCH₂CH₂SO₃H, R₄= α-Me, R₅= (*S,R*)Me
**Ih4:** R₁= α-OH, R₂= α-OH, R₃= NECH₂CH₂SO₃H, R₄= α-Me, R₅= (*S*)Me
**Ii4 :** R₁= α-OH, R₂= α-OH, R₃= NHCH₂CH₂SO₃H, R₄= α-Me, R₅= (*R*)Me
**Il4:** R₁= β-OH, R₂= α-OH, R₃= NHCH₂CH₂SO₃H, R₄= α-Me, R₅= (*S,R*)Me
**Im4:** R₁= β-OH, R₂= α-OH, R₃= NHCH₂CH₂SO₃H, R₄= α-Me, R₅= (*S*)Me
**In4:** R₁= β-OH, R₂= α-OH, R₃= NHCH₂CH₂SO₃H, R₄= α-Me, R₅= (*R*)Me
**Ia5:** R₁= α-OH, R₂= H, R₃= NHCH₂CO₂H, R₄= α-Me, R₅= (*S,R*)Me
**Ib5:** R₁= α-OH, R₂= H, R₃= NHCH₂CO₂H, R₄= α-Me, R₅= (*S*)Me
**Ic5:** R₁= α-OH, R₂= H, R₃= NHCH₂CO₂H, R₄= α-Me, R₅= (*R*)Me
**Id5:** R₁= β-OH, R₂= H, R₃= NHCH₂CO₂H, R₄= α-Me, R₅= (*S,R*)Me
**Ie5:** R₁=β-OH, R₂=H, R₃=NHCH₂CO₂H, R₄=α-Me, R₅= (*S*)Me
**If5:** R₁=β-OH, R₂=H, R₃=NHCH₂CO₂H, R₄=α-Me, R₅=(*R*)Me
**Ig5:** R₁=α-OH, R₂=α-OH, R₃=NHCH₂CO₂H, R₄=α-Me, R₅=(*S*,*R*)Me
**Ih5:** R₁=α-OH, R₂=α-OH, R₃=NHCH₂CO₂H, R₄=α-Me, R₅=(*S*)Me
**Ii5:** R₁=α-OH, R₂=α-OH, R₃=NHCH₂CO₂H, R₄=α-Me, R₅=(*R*)Me
**Il5:** R₁=β-OH, R₂=α-OH, R₃=NHCH₂CO₂H, R₄=α-Me, R₅=(*S*,*R*)Me
**Im5:** R₁=β-OH, R₂=α-OH, R₃=NHCH₂CO₂H, R₄=α-Me, R₅=(*S*)Me
**In5:** R₁=β-OH, R₂=α-OH, R₃=NHCH₂CO₂H, R₄=α-Me, R₅=(*R*)Me

All publications and patent documents cited herein are incorporated herein by reference as if each such publication or document was specifically and individually indicated to be incorporated herein by reference. Citation of publications and patent documents is not intended as an admission that any is pertinent prior art, nor does it constitute any admission as to the contents or date of the same. The invention having now been described by way of written description, those of skill in the art will recognize that the invention can be practiced in a variety of embodiments and that the foregoing description and examples below are for purposes of illustration and not limitation of the claims that follow.

### EXAMPLE 1: Synthesis of TGRS Modulators

The compounds of the invention, and related derivatives, can be synthesized by methods known to one skilled in the art. Detailed methods for synthesizing these compounds are described below. *See, also,* WO 02/072598, WO 2004/0007521, EP 1568706 and EP 135782. In the case of the compound where R₁ and R₃ are hydrogen, R₂ is hydroxy and R₄ is a lower alkyl group, the compound of formula (I) can be obtained in accordance with the following scheme: 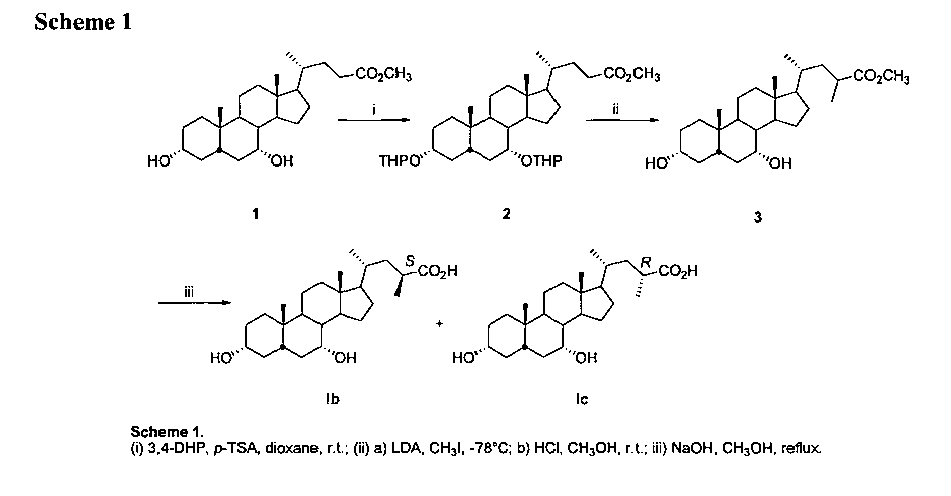 Methyl chenodeoxycholanoate (**1**) was protected in 3- and 7-position by treatment with 3,4-dihydro-2H-pyran in dioxane in presence of catalytic amount of *p*-toluenesulfonic acid (p-TSA) to give the corresponding 3α,7α-tetrahydropyranyloxy analog (**2**). Reaction of **2** with methyl iodide (or with an appropriate alkyl halide), at -78 °C using lithium diisopropylamide as a base and tetrahydrofuran (THF) as solvent, followed by treatment with methanolic HCl afforded the corresponding methyl 23-methyl-3α,7α-dihydroxy-5β-cholan-24-oate (**3**). Hydrolysis with alkali of the methyl ester **3** and purification by flash chromatography yielded the desired 23(S)-methyl-3α,7α-dihydroxy-5β-cholan-24-oic acid (**Ib**) and 23(*R*)-methyl-3α,7α-dihydroxy-5β-cholan-24-oic acid (**Ic**).

### Preparation of 23(R)- and 23(S)-methyl-3α,7α-dihydroxy-5β-cholan-24-oic acid (Ib, Ic)

### a) Methyl 3α,7α-ditetrahydropyranyloxy-5β-cholan-24-oate (2)

p-Toluenesulfonic acid (78 mg, 0.41 mmol), 3,4-dihydro-2H-pyrane (20.1 ml, 0.098 mol) were added to a solution of methyl 3α,7α-dihydroxy-5β-cholan-24-oate (**1**) (2.0 g, 4.9 mmol) in dioxane (6 mL). The reaction mixture was stirred at room temperature for 15 min. H₂O (50 mL) was then added and the mixture was partially concentrated under vacuum and extracted with EtOAc (3 x 50 mL). The combined organic fractions were washed with brine (1 x 50 mL), dried (Na₂SO₄) and evaporated under vacuum. The residue was purified by chromatography on silica gel column. Elution with light petroleum/ethyl acetate 80/20 afforded 2.5 g of the pure compound 2 (90% yield).
¹H-NMR (CDCl₃) δ: 0.64 (s, 3H, CH₃-18), 0.89 (s, 3H, CH₃-19), 0.92 (d, 3H, CH₃-21), 3.31-3.67 (m, 4H, -C*H₂*OCH)- 3.65 (s, 3H, CO₂C*H₃*), 3.67 (m, 1H, CH-3), 3.88 (brs, 1H, CH-7), 4.67 (brs, 1H, -O-CH-O-), 4.73 (brs, 1H, -O-CH-O-).

### b) Methyl 23(R,S)-methyl-3α,7α-dihydroxy-5β-cholan-24-oate (3)

n-Butyl lithium (4.3 mL, 2.2 M solution in hexane) were added dropwise at -78 °C to a solution of diisopropylamine (1.4 mL, 10.1 mmol) in dry THF (50 mL). The system was kept to -78 °C for additional 30 min and then, methyl 3α,7α,12α-tetrahydropyranyloxy-5β-cholan-24-oate (2) (1.8 g, 3.2 mmol) dissolved in dry THF (14 mL) was added dropwise to the mixture. After 20 min methyl iodide (1.4 mL, 22.0 mmol) dissolved in dry THF (7 mL) was slowly added and the mixture was allowed to warm to room temperature overnight. The solvents were removed under vacuum and acidified by 10% HCl and extracted with EtOAc (5 x 50 mL), washed with 5% Na₂S₂O₃ solution (2 x 50 mL), dried (over anhydrous Na₂SO₄), filtered, and evaporated under vacuum. The crude residue was then treated with a solution of 2N HCl in MeOH (50 mL) for 12 h. The residue was evaporated under vacuum and taken up with EtOAc (100 mL), washed with a saturated NaHCO₃ solution (2 x 50 mL), dried (Na₂SO₄) and evaporated under vacuum. The residue was purified by silica gel flash chromatography. Elution with light petroleum/ethyl acetate 70/30 afforded 1.1 g (2.7 mmol) of the pure compound **3** (84% yield).
¹H-NMR (CDCl₃) δ : 0.62 (s, 3H, CH₃-18), 0.87 (s, 3H, CH₃-19), 0.92 (d, 3H, CH₃-21), 2.38 (m, 1H, CH-23), 3.27-3.40 (m, 1H, CH-3), 3.55 (brs, 1H, CH-7), 3.63 (s, 3H, CO₂C*H*₃).

### c) 23(R)-Methyl-3α,7α-dihydroxy-5β-cholan-24-oic acid (Ib) and 23(S)-Methyl-3α,7α-dihydroxy-5β-cholan-24-oic acid (Ic)

Methyl 23-methyl-3α,7α-dihydroxy-5β-cholan-24-oate 0.97 g (2.3 mmol) was dissolved in MeOH (25 mL) and added with 10% NaOH in MeOH (5.7 mL, 14.2.mmol). The mixture was refluxed for 16 h. The mixture was acidified with 3N HCl and extracted with EtOAc (3 x 20 mL). The combined organic fractions were washed with brine (1 x 50 mL), dried (Na₂SO₄) and evaporated under vacuum. The residue was purified by silica gel flash chromatography. Elution with CHCl₃:MeOH (95/5) afforded 1.5 g (65%) of 23(*S*)-Methyl-3α,7α-dihydroxy-5β-cholan-24-oic acid and 330 mg of 23(R)-Methyl-3α,7α-dihydroxy-5β-cholan-24-oic acid.

23(*S*)-Methyl-3α,7α-dihydroxy-5β-cholan-24-oic acid **(Ib):** mp: 125-126 °C. ¹H-NNM (CDCl₃+CD₃OD) δ: 0.44 (s, 3H, CH₃-18), 0.69 (s, 3H, CH₃-19), 0.73-0.76 (d, 3H CH₃-21), 0.93-0.97 (d, 3H, -CH₃), 2.36 (m, 1H, CH-23), 3.15-3.38 (m, 1H, CH-3), 3.62 (brs, 1H, CH-7). ¹³C-NMR (CDCl₃+CD₃OD) δ: 11.55, 18.43, 18.87, 20.49, 22.69, 28.15, 28.57, 30.14, 32.65, 34.43, 34.61, 34.94, 35.23, 37.06, 39.17, 39.60, 40.81, 41.40, 42.57, 46.54, 50.29, 56.63, 68.24, 71.62, 179.99.

23(*R*)-Methyl-3α,7α-dihydroxy-5β-cholan-24-oic acid (**Ic**) : mp: 163-164 °C. ¹H-NMR (CDCl₃+CD₃OD) δ : 0.43 (s, 3H, CH₃-18), 0.65 (s, 3H, CH₃-19), 0.65-0.69 (d, 3H CH₃-21), 0.83-0.86 (d, 3H, -CH₃), 2.20 (m, 1H, CH-23), 3.09-3.15 (m, 1H, CH-3), 3.58 (brs, 1H, CH-7). ¹³C-NMR (CDCl₃+CD₃OD) δ : 11.94, 16.40, 18.30, 20.93, 23.06, 23.89, 28.85, 30.52, 33.08, 34.16, 34.91, 35.38, 35.68, 37.14, 39.49, 39.64, 40.04, 40.17, 41.92, 43.05, 50.69, 57.10, 68.51, 72.01, 181,09.

The following compounds were prepared by alkylation of 6α-methyl-3α,7α-dihydroxy-5β-cholan-24-oic acid according to the procedure of Example 1.

### EXAMPLE 2: Preparation of 23(S)- and 23(R)-methyl-6α-methyl-3α,7α-dihydroxy-5β-cholan-24-oic acid (Ib3, Ic3)

23(*S*)-Methyl-6α-methyl-3α,7α-dihydroxy-5β-cholan-24-oic acid (**Ib3**): mp: 98-100 °C. ¹H-NMR (CDCl₃) δ: 0.63 (s, 3H, CH₃-18), 0.89 (s, 3H, CH₃-19), 0.92-1.00 (m, 6H, CH₃-21 and CH₃-6), 1.15-1.19 (d, 3H, -CH₃), 2.45-2.73 (m, 1H, CH-23), 3.31-3.52 (m, 1H, CH-3), 3.58 (brs, 1H, CH-7). ¹³C-NMR (CDCl₃) δ: 11.76, 15.72, 18.58, 18.88, 20.63, 23.11, 23.65, 28.19, 30.21, 30.47, 32.64, 33.79, 33.97, 34.61, 35.42, 35.66, 37.03, 39.60, 40.01, 40.71, 42.71, 47.35, 50.44, 56.60, 72.34, 72.87, 182.37.

23(*R*)-Methyl-3α,7α-dihydroxy-5β-cholan-24-oic acid (**Ic3**): mp: 89-90 °C. ¹H-NMR (CDCl₃+CD₃OD) δ: 0.65 (s, 3H, CH₃-18), 0.88 (s, 3H, CH₃-19), 0.88-0.92 (m, 3H, CH₃-6), 0.95-0.99 (d, 3H, CH₃-21), 1.08-1.14 (d, 3H-CH₃), 2.35 (m, 1H, CH-23), 3.29-3.48 (m, 1H, CH-3), 3.57 (brs, 1H, CH-7). ¹³C-NMR (CDCl₃+CD₃OD) δ: 11.70, 15.66, 16.02, 18.00, 20.61, 23.09, 23.60, 28.51, 30.39, 32.61, 33.72, 33.92, 35.38, 35.65, 36.33, 39.57, 39.94, 42.77, 47.30, 50.39, 56.53, 72.22, 72.83, 180.50.

The following compounds were prepared by alkylation of 3α,7α,12α-trihydroxy-5β-cholan-24-oic acid according to the procedure of Example 1.

### EXAMPLE 3: Preparation of 23(R)- and 23(S)-methyl-3α,7α,12α-trihydroxy-5β-cholan-24-oic acid (Ih, Ii)

23(*S*)-Methyl-3α,7α,12α-trihydroxy-5β-cholan-24-oic acid **(Ih):** mp: 237-239 °C. ¹H-NMR (CDCl₃) δ: 0.63 (s, 3H, CH₃-18), 0.87 (s, 3H, CH₃-19), 0.96-0.98 (m, 3H, CH₃-21), 1.07-1.11 (d, 3H, -CH₃), 2.44-2.73 (m, 1H, CH-23), 3.35-3.50 (m, 1H, CH-3), 3.82 (brs, 1H, CH-7) 3.95 (brs, 1H, CH-12). ¹³C-NMR (DMSO) δ: 12.72, 17.60, 19.24, 19.24, 23.00, 23.19, 26.59,27.78, 28.88, 30.72, 34.77, 35.22, 35.66, 37.19, 41.84, 46.19, 47.27, 49.01, 66.69, 70.88, 71.45, 178.25.

23(*R*)-Methyl-3α,7α,12α-trihydroxy-5β-cholan-24-oic acid **(Ii):** mp: 221-223 °C. ¹H-NMR (CDCl₃) δ: 0.63 (s, 3H, CH₃-18), 0.87 (s, 3H, CH₃-19), 0.96-0.98 (m, 3H, CH₃-21), 1.07-1.11 (d, 3H, -CH₃), 2.44-2.73 (m, 1H, CH-23), 3.35-3.50 (m, 1H, CH-3), 3.82 (brs, 1H, CH-7) 3.95 (brs, 1H, CH-12). ¹³C-NMR (DMSO) δ: 12.76, 16.88, 17.31, 23.04, 23.24, 26.62, 28.12, 28.94, 30.81, 33.97, 34.80, 35.28, 35.71, 37.20, 41.85, 46.29, 47.44, 66.67, 70.86, 71.45, 178.77.

The following compounds were prepared by alkylation of 6α-methyl-3α,7α,12α-trihydroxy-5β-cholan-24-oic acid according to the procedure of Example 1.

### EXAMPLE 4: Preparation of 23(R)- and 23(S)-methyl-6α-methyl-3α,7α,12a-trihydroxy-5β-cholan-24-oic acid (Ih3, Ii3)

23(*S*)-Methyl-6α-methyl-3α,7α,12α-trihydroxy-5β-cholan-24-oic acid (**Ih3**): mp: 131-134 °C. ¹H-NMR (CDCl₃+CD₃OD) δ: 0.65 (s, 3H, CH₃-18), 0.87 (s, 3H, CH₃-19), 0.97-1.00 (m, 3H, CH₃-21), 1.14-1.18 (d, 3H, -CH₃), 1.23 (m, 1H, CH-6), 2.52 (m, 1H, CH-23), 3.32-3.50 (m, 1H, CH-3), 3.55 (brs, 1H, CH-7) 3.94 (brs, 1H, CH-12). ¹³C-NMR (CDCl₃+CD₃OD) δ: 12.43, 145.66, 17.62, 18.92, 22.70, 23.14, 26.21, 27.45, 28.01, 30.03, 33.44, 34.11, 34.42, 35.30, 36.71, 39.97, 40.45, 41.73, 46.45, 47.25, 72.13, 72.76, 73.01,180.53.

23(*R*)-Methyl-6a-methyl-3α,7α,12α-trihydroxy-5β-cholan-24-oic acid (**Ii3**): mp: 109-110 °C. ¹H-NMR (CD₃OD) δ: 0.72 (s, 3H, CH₃-18), 0.91 (s, 3H, CH₃-19), 1.07-1.11 (m, 6H, -CH₃ and CH₃-21), 2.37-2.53 (m, 1H, CH-23), 3.15-3.42 (m, 1H, CH-3), 3.53 (brs, 1H, CH-7) 3.97 (brs, 1H, CH-12). ¹³C-NMR (CD₃OD) δ: 11.61, 15.04, 15.32, 16.15, 22.04, 22.75, 26.27, 27.62, 28.18, 29.61, 32.91, 33.74, 34.31, 35.06, 35.18, 36.56, 39.70, 40.25, 41.68, 46.19, 46.31, 71.76, 71.77, 72.62, 180.11.

The following compounds were prepared by alkylation of 3α-hydroxy-5β-cholan-24-oic acid according to the procedure of Example 1.

### Example 4: Preparation of 23(R)- and 23(S)-methyl-3α-hydroxy-5β-cholan-24-oic acid (Ip, Iq)

23(*S*)-Methyl-3α-hydroxy-5β-cholan-24-oic acid (**Ip**): mp: 161-162 °C. ¹H-NMR (CDCl₃+CD₃OD) δ: 0.60 (s, 3H, CH₃-18), 0.88 (s, 3H, CH₃-19), 0.92-1.01 (m, 3H, CH₃-21), 1.13-1.16 (d, 3H, -CH₃), 2.55 (m, 1H, CH-23), 3.60 (m, 1H, CH-3). ¹³C-NMR (CDCl₃+CD₃OD) δ : 11.97, 18.52, 18.87, 20.73, 23.30, 24.14, 26.34, 27.10, 28.15, 30.18, 34.48, 34.50, 35.23, 35.74, 36.06, 37.01, 40.13, 40.34, 40.74, 41.99, 42.68, 56.43, 56.75, 71.70, 181.42.

23(*R*)-Methyl-3α-hydroxy-5β-cholan-24-oic acid (**Iq**): mp: 152-153 °C. ¹H-NMR (CDCl₃+CD₃OD) δ: 0.63 (s, 3H, CH₃-18), 0.89 (s, 3H, CH₃-19), 0.94-1.03 (m, 3H, CH₃-21), 2.45 (m, 1H, CH-23), 3.59 (m, 1H, CH-3). ¹³C-NMR (CD₃OD) δ: 11.98, 15.97, 18.00, 20.75, 23.31, 24.14, 26.34, 27.11, 28.48, 30.26, 33.68, 34.50, 35.26, 35.77, 36.15, 36.46, 39.59, 40.13, 40.36, 42.01, 42.79, 56.45, 56.76, 71.71,181.02.

### EXAMPLE 5: Biological Activity

The potency and efficacy of the compounds of the invention on TGR5 receptor was evaluated using in vitro assays. See, Kawamata, J. Biol. Chem 2003, Vol. 278 No. 11, p. 9435-9440). Activity on FXR was assayed by fluorescence resonance energy transfer (FRET) for recruitment of the SRC-1 peptide to human FXR using a cell-free ELiSA. See, Blanchard et al. WO 00/37077.

**Table 1. EC₅₀ (µM) of Example Compounds on FXR and TGR5 Receptor**

| **Compound** | **Structure** | **FXR data** | **TGR5 Data** |
|---|---|---|---|
| **CDCA** **(ChenoDeoxyCholicAcid)** | 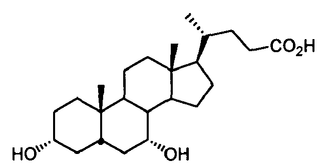 | EC₅₀: 8.6 µM | EC₅₀: 4.0 µM |
| | | Efficacy: 100% | Efficacy: 100% |
| **6α-MeCDCA** | 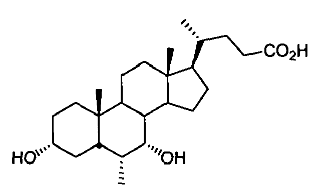 | EC₅₀: 0.21 µM | EC₅₀: 0.37 µM |
| | | Efficacy: 148% | Efficacy: 119% |
| **23(*R*+*S*)-Me -6MeCDCA (I3a)** | 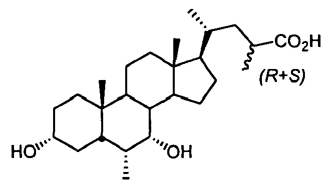 | EC₅₀: 15.62 µM | EC₅₀: 0.11 µM |
| | | Efficacy: 60% | Efficacy: 123% |

The compounds of the invention are potent and selective TGR5 modulators. The introduction of an alkyl group at the C-23 position of bile acid gives selectivity for the TGR5 receptor with respect to FXR. This is evident by the observation of the biological results obtained for CDCA, 6-MeCDCA and 6,23-diMe-CDCA (23-*R,S* isomers mixture) on FXR and TGR5 as shown in Table 1. 6,23-diMe-CDCA is 100-fold more potent on TGR5 with respect to the FXR receptor.

### Other Embodiments

While the invention has been described in conjunction with the detailed description thereof, the foregoing description is intended to illustrate and not limit the scope of the invention, which is defined by the scope of the appended claims. Other aspects, advantages, and modifications are within the scope of the following claims. It will be understood by those skilled in the art that various changes in form and details may be made therein without departing from the scope of the invention encompassed by the appended claims.

## Claims

**1.** A compound of Formula I: 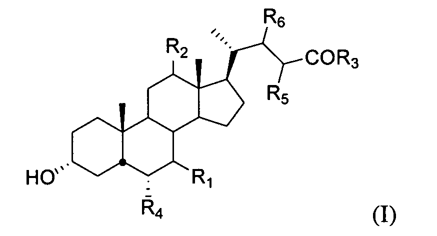 or a pharmaceutically effective salt, solvate, hydrate, or prodrug thereof, wherein:
R₁ is hydrogen, hydroxy, or halogen;
R₂ is hydrogen or α-hydroxy;
R₃ is hydroxy, NH(CH₂)ₘSO₃H, or NH(CH₂)ₙCO₂H;
R₄ is hydrogen, alkyl, or halogen;
R₅ is unsubstituted or substituted alkyl, or aryl;
R₆ is hydrogen or R₅ and R₆ taken together with the carbons to which they are attached form a ring of size 3, 4, 5, or 6 atoms;
m is an integer 0, 1, 2, 3, 4, or 5; and
n is an integer 0, 1, 2, 3, 4, or 5;
with the proviso that when R₅ is methyl, R₁ is hydroxyl, and R₃ is hydrogen or NHCH₂CH₂SO₃H, then R₄ is not hydrogen.

**2.** The compound of claim 1, wherein R₁ is hydrogen or hydroxy.

**3.** The compound of claim 2, wherein R₁ is hydroxy.

**4.** The compound of claim 2, wherein R₁ is hydrogen.

**5.** The compound of claim 3, wherein R₁ is α-hydroxy.

**6.** The compound of claim 3, wherein R₁ is β-hydroxy.

**7.** The compound of claim 1, wherein R₂ is α-hydroxy.

**8.** The compound of claim 6, wherein R₁ is β-hydroxy and R₂ is α-hydroxy.

**9.** The compound of claim 6, wherein R₁ is β-hydroxy and R₂ is H.

**10.** The compound of claim 5, wherein R₁ is α-hydroxy and R₂ is H.

**11.** The compound of claim 1, wherein at least one of R₁ or R₂ is hydroxy.

**12.** The compound of claim 1, wherein at least one of R₁ or R₂ is hydrogen.

**13.** The compound of claim 1, wherein R₁ and R₂ are the same.

**14.** The compound of claim 5, wherein R₁ and R₂ are each α-hydroxy.

**15.** The compound of claim 4, wherein R₁ and R₂ are each hydrogen.

**16.** The compound of claim 1, wherein R₃ is hydrogen, NH(CH₂)ₘSO₃H, or NH(CH₂)ₙCO₂H.

**17.** The compound of claim 16, wherein R₃ is hydrogen.

**18.** The compound of claim 16, wherein R₃ is not hydrogen.

**19.** The compound of claim 16, wherein R₃ is NH(CH₂)ₘSO₃H.

**20.** The compound of claim 19, wherein m is 2.

**21.** The compound of claim 16, wherein R₃ is NH(CH₂)ₙCO₂H.

**22.** The compound of claim 21, wherein n is 1.

**23.** The compound of claim 1, wherein R₄ is hydrogen or alkyl.

**24.** The compound of claim 23, wherein R₄ is hydrogen.

**25.** The compound of claim 23, wherein R₄ is alkyl.

**26.** The compound of claim 25, wherein R₄ is in the alpha configuration.

**27.** The compound of claim 25, wherein R₄ is methyl or ethyl.

**28.** The compound of claim 27, wherein R₄ is methyl.

**30.** The compound of claim 27, wherein R₄ is ethyl.

**32.** The compound of claim 1, wherein R₃ and R₄ are the same.

**33.** The compound of claim 1, wherein R₃ and R₄ are different.

**34.** The compound of claim 32, wherein R₃ and R₄ are each hydrogen.

**35.** The compound of claim 33, wherein R₃ is NH(CH₂)ₘSO₃H and R₄ is hydrogen.

**36.** The compound of claim 35, wherein m is 2.

**37.** The compound of claim 33, wherein R₃ is NH(CH₂)ₙCO₂H and R₄ is hydrogen.

**38.** The compound of claim 37, wherein n is 1.

**39.** The compound of claim 33, wherein R₃ is H and R₄ is alkyl.

**40.** The compound of claim 39, wherein alkyl is in the alpha configuration.

**41.** The compound of claim 39, wherein R₄ is methyl.

**42.** The compound of claim 39, wherein R₄ is ethyl.

**43.** The compound of claim 39, wherein R₄ is α-methyl.

**45.** The compound of claim 1, wherein R₅ is unsubstituted or substituted alkyl.

**46.** The compound of claim 1, wherein R₅ is in the S-configuration.

**47.** The compound of claim 1, wherein R₅ is in the R-configuration.

**48.** The compound of claim 45, wherein R₅ is methyl or ethyl.

**49.** The compound of claim 48, wherein R₅ is S-methyl.

**50.** The compound of claim 48, wherein R₅ is R-methyl.

**51.** The compound of claim 48, wherein R₅ is S-ethyl.

**52.** The compound of claim 48, wherein R₅ is R-ethyl.

**53.** The compound of claim 45, wherein R₅ is substituted with phenyl.

**54.** The compound of claim 53, wherein R₅ is benzyl.

**55.** The compound of claim 54, wherein R₅ is S-benzyl.

**56.** The compound of claim 54, wherein R₅ is R-benzyl.

**57.** The compound of claim 1, wherein R₅ is aryl.

**58.** The compound of claim 57, wherein R₅ is phenyl.

**59.** The compound of claim 1, wherein R₄ and R₅ are each alkyl.

**60.** The compound of claim 59, wherein R₅ is in the S-configuration.

**62.** The compound of claim 59, wherein R₄ is in the alpha-configuration.

**63.** The compound of claim 59, wherein R₁ is α-hydroxy.

**64.** The compound of claim 59, wherein R₂ is hydrogen.

**65.** The compound of claim 1, wherein R₄ and R₅ are each alkyl, R₁ is α-hydroxy, and R₂ is hydrogen.

**66.** The compound of claim 1, wherein R₁, R₂, R₃, and R₄ are hydrogen.

**67.** The compound of claim 1, wherein R₂, R₃, and R₄ are hydrogen.

**68.** The compound of claim 1, wherein R₂ and R₃ are hydrogen.

**69.** The compound of claim 1, wherein at least one of R₁, R₂, R₃, R₄, or R₅ is hydrogen.

**70.** The compound of claim 1, wherein at least two of R₁, R₂, R₃, R₄, or R₅ is hydrogen.

**71.** The compound of claim 1, wherein at least three of R₁, R₂, R₃, R₄, or R₅ is hydrogen.

**72.** The compound of claim 1, wherein at least four of R₁, R₂, R₃, R₄, or R₅ is hydrogen.

**73.** The compound of claim 1, wherein the compound is selected from Compounds **Ia, Ib, Ic, Ig, Ih, Ii, Io, Ip, Iq, Ia1, Ib1, Ic1, Ig1, Ih1, Iil, Il1, Im1, In1, Io1, Ip1, Iq1, Ia2, Ib2, Ic2, Id2, Ie2, If2, Ig2, Ih2, Ii2, Il2, Im2, In2, Io2, Ip2, Iq2, Ia3, Ib3, Ic3, Id3, Ie3, If3, Ig3, Ih3, Ii3, Il3, Im3, In3, Ia4, Ib4, Ic4, Id4, Ie4, If4, Ig4, Ih4, Ii4, Il4, Im4, In4, Ia5, Ib5, Ic5, Id5, Ie5, If5, Ig5, Ih5, Ii5, Il5, Im5** or **In5.**

**74.** A composition comprising a compound of formula I: 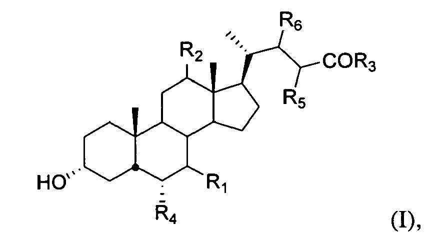 or a salt, solvate, hydrate, or prodrug thereof, and at least one pharmaceutically acceptable excipient wherein:
R₁ is hydrogen, hydroxy, or halogen;
R₂ is hydrogen or α-hydroxy;
R₃ is hydroxy, NH(CH₂)ₘSO₃H, or NH(CH₂)ₙCO₂H;
R₄ is hydrogen, alkyl, or halogen;
R₅ is unsubstituted or substituted alkyl, or aryl;
R₆ is hydrogen or R₅ and R₆ taken together with the carbons to which they are attached form a ring of size 3, 4, 5, or 6 atoms;
m is an integer 0, 1, 2, 3, 4, or 5; and
n is an integer 0, 1, 2, 3, 4, or 5;
with the proviso that when R₅ is methyl, R₁ is hydroxyl, and R₃ is hydrogen or NHCH₂CH₂SO₃H, then R₄ is not hydrogen.

**75.** A method of treating disease in a subject, comprising administering a therapeutically effective amount of a compound of formula I to a subject in need thereof: 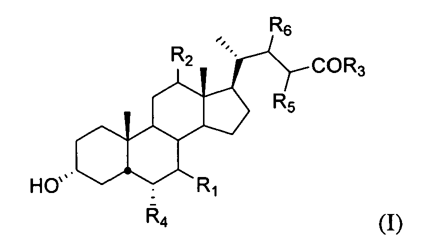 or a pharmaceutically effective salt, solvate, hydrate, or prodrug thereof, wherein:
R₁ is hydrogen, hydroxy, or halogen;
R₂ is hydrogen or α-hydroxy;
R₃ is hydroxy, NH(CH₂)ₘSO₃H, or NH(CH₂)ₙCO₂H;
R₄ is hydrogen, alkyl, or halogen;
R₅ is unsubstituted or substituted alkyl, or aryl;
R₆ is hydrogen or R₅ and R₆ taken together with the carbons to which they are attached form a ring of size 3, 4, 5, or 6 atoms;
m is an integer 0, 1, 2, 3, 4, or 5; and
n is an integer 0, 1, 2, 3, 4, or 5.

**75.** The method of claim 74, wherein treatment of the disease involves modulation of TGR5 receptor.

**76.** The method of claim 75, wherein the disease is obesity.

**77.** The method of claim 75, wherein the disease is insulin sensitivity.

**78.** The method of claim 75, wherein the disease is inflammation.

**79.** The method of claim 75, wherein the subject is human.

**80.** The method of claim 75, wherein when R₅ is methyl; R₁ is hydroxy; R₂ is hydrogen or NHCH₂CH₂SO₃H, then R₄ is not hydrogen.

**81.** The method of claim 75, wherein the compound is selected from Compounds **Ia, Ib, Ic, Id, Ie, If, Ig, Ih, Ii, Il, Im, In, Io, Ip, Iq, Ia1, Ib1, Ic1, Id1, Ie1, If1, Ig1, Ih1, Iil, Il1, Im1, In1, Io1, Ip1, Iq1, Ia2, Ib2, Ic2, Id2, Ie2, If2, Ig2, Ih2, Ii2, Il2, Im2, In2, Io2, Ip2, Iq2, Ia3, Ib3, Ic3, Id3, Ie3, If3, Ig3, Ih3, Ii3, I13, Im3, In3, Ia4, Ib4, Ic4, Id4, Ie4, If4, Ig4, Ih4, Ii4, Im4, In4, Ia5, Ib5, Ic5, Id5, Ie5, If5, Ig5, Ih5, Ii5, Il5, Im5 or In5.**

**82.** The method of claim 81, wherein the compound is selected from Compounds **Ia, Ib, Ic, Ig, Ih, Ii, Io, Ip, Iq, Ia1, Ib1, Ic1, Ig1, Ih1, Iil, Il1, Im1, In1, lo1, Ip1, Iq1, Ia2, Ib2, Ic2, Id2, Ie2, If2, Ig2, Ih2, Ii2, Il2, Im2, In2, Io2, Ip2, Iq2, Ia3, Ib3, Ic3, Id3, Ie3, If3, Ig3, Ih3, Ii3, Il3, Im3, In3, Ia4, Ib4, Ic4, Id4, Ie4, If4, Ig4, Ih4, Ii4, Il4, Im4, In4, Ia5, Ib5, Ic5, Id5, Ie5, If5, Ig5, Ih5, Ii5, Il5, Im5 or In5.**

**83.** The method of claim 74, wherein the compound is a compound of formula I with the proviso that when R₅ is methyl, R₁ is hydroxyl, and R₃ is hydrogen or NHCH₂CH₂SO₃H, then R₄ is not hydrogen.

**84.** A method of treating disease in a subject, comprising administering a therapeutically effective amount of the compound of claim 1 to a subject in need thereof.

**85.** The method of claim 84, wherein the disease is selected from cholestasis or bile desaturation.
